# EUROPEAN PATENT SPECIFICATION

(11) **EP 3 518 888 B1**
(45) Date of publication and mention of the grant of the patent: **19.11.2025**
(21) Application number: 17780047.1
(22) Date of filing: 25.09.2017
(51) Int. Cl.: A61K 8/49, A61Q 3/00, A61K 8/97

(54) **ALKYL-PHTALIDES FOR USE IN THE COSMETIC TREATMENT OF NAILS**
ALKYL-PHTHALIDE ZUR VERWENDUNG BEI DER KOSMETISCHEN BEHANDLUNG VON NÄGELN
ALKYLPHTALIDES DESTINÉS À ÊTRE UTILISÉS DANS LE TRAITEMENT COSMÉTIQUE DES ONGLES

(30) Priority: 27.09.2016 FR 1659081
(43) Date of publication of application: 07.08.2019
(73) Proprietor: SEDERMA, 78610 Le-Perray-en-Yvelines (FR)
(72) Inventor: BONDIL, Céline, 78610 Le Perray-en-Yvelines (FR); DORIDOT, Emmanuel, 78610 Le Perray-en-Yvelines (FR); MONDON, Philippe, 78610 Le Perray-en-Yvelines (FR)
(74) Representative: Munier, Mélanie
(86) International application number: PCT/EP2017/074262
(87) International publication number: WO 2018/060147

(56) References cited:
- WO-A1-2016/157073
- CN-A- 103 183 654
- CN-A- 107 396 627
- FR-A1- 2 827 509
- FR-A1- 2 982 150
- US-B1- 6 203 782
- DATABASE GNPD [online] MINTEL; 31 December 2012 (2012-12-31), "Naturally Enriching Hand & Nail Cream", XP002766494, Database accession no. 1937059
- LUND E D: "FLAVORS AND NONALCOHOLIC BEVERAGES. ÖTHIN LAYER AND HIGH PRESSURE LIQUID CHROMATOGRAPHIC ANALYSIS OF CELERY SEED OIL", JOURNAL OF THE ASSOCIATION OF OFFICIAL ANALYTICAL CHEMISTS, THE ASSOCIATION, ARLINGTON, VA, US, vol. 61, no. 5, 1 January 1978 (1978-01-01), pages 1083 - 1088, XP000563665, ISSN: 0004-5756

## Description

### TECHNICAL FIELD

The present invention relates to the cosmetic treatment of nails.

Cosmetics, hygiene and personal care, and dermo-pharmacy industries are concerned, for human and animals.

The aim of the present invention is, in particular, to propose a means for preventing or treating weaken nails, which most characteristic signs are the fact that the nails are soft, brittle and/or split, grow badly and slowly, and less beautiful because they are not smooth, having longitudinal ridges.

### BACKGROUND ART

The *Apium graveolens* plant, commonly called celery, is part of the family of Apiaceae or Umbelliferae family, which includes many other edible plants such as carrot and coriander. This plant is not toxic; all parts of it can be consumed as raw or cooked vegetables. The seeds are also used as spices.

Italy is the country of origin, this plant being widespread in the world, especially in Europe, Egypt, Algeria, Ethiopia, Asia and India.

Celery seeds contain about 3% of volatile oil and 15% of non-volatile vegetable oil.

An essential oil extracted from celery seeds comprises terpenes (D-limonene > 60% and selinene 10-20%) and phthalides (1-4%). Among the predominant phthalides, there is the 3-n-butyl-phthalide, the sedanolide and sedanenolide (also called senkyunenolide A).

The nonvolatile part includes petroselinic, oleic, linoleic, myristic, palmitic, palmitoleic, stearic and myristoleic fatty acids.

The *Apium graveolens* therefore comprises potentially very many active compounds. As a medicinal plant, it is recommended for thousands of years for many properties: aphrodisiac, anthelmintic, antispasmodic, anti-inflammatory, carminative, diuretic, emmenagogue, laxative, sedative, stimulant and tonic. The plant can be used against asthma, bronchitis and rheumatism. The wild celery seeds are used in traditional medicine in India and other countries that have adopted the Ayurvedic traditions to heal, as a tranquilizer, antispasmodic, nerve tonic, diuretic and antirheumatic. The phthalides included in the *Apium graveolens* are described as active against cancer, high blood pressure and cholesterol.

The world of living organisms and plants in particular represents a very important source of innovative active molecules for cosmetics and pharmaceuticals. To extract and concentrate them, there are different methods, and the composition of the obtained extract will obviously depend on the method and the operating conditions.

The extraction technique with supercritical CO₂ is already widely used in the food, pharmaceutical and perfumery industries. Gaseous CO₂, present in low concentrations in the air, is compressed so that it becomes liquid. It is then an excellent solvent for fat-soluble molecules. Inert, it reacts little with the extracted molecules and leaves no residue since it is evaporated at the end of process and captured to be reused.

An essential oil of celery, obtained by hydro-distillation, comprising in majority D-limonene has been proposed in cosmetics to treat pigmentation spots.

The database GNPD MINTEL (2012-12-31), XP002766494, Database accession no. 1937059, discloses a "Naturally Enriching Hand & Nail Cream" that comprises essential oils of *Apium Graveolens* seeds. CN103183654 discloses a CO₂ supercritical extraction method of *Apium Graveolens* seeds for recovering flavonoids and discloses the known general properties of flavonois.

### SUMMARY OF THE INVENTION

The present invention provides the cosmetic non-therapeutic topical use of an extract of *Apium graveolens* seeds prepared by CO₂ supercritical extraction for the topical treatment of nails, said extract comprising at least 50 % by weight of alkyl-phthalide mixture constituted of sedanenolide, sedanolide and 3-n-butylphthalide, in particular for the treatment of soft, brittle, split nails and/or nails having longitudinal ridges.

The present invention also provides a varnish product according to claim 8.

It is also disclosed herein a product for a cosmetic nail treatment of the invention in the form a patch, dissolving, adhesive, woven or non-woven product comprising at least one alkyl-phthalide or a plant extract comprising said alkyl-phthalide as active component in a varnish adapted matrix/excipient, preferably said alkyl phthalide being sedanenolide, more preferably said plant extract being a CO₂ supercritical extract of seeds of *Apium graveolens* comprising a mixture of alkyl-phthalides constituted of sedanenolide, sedanolide and 3-n-butylphthalide.

"Cosmetic treatment" means a treatment that addresses normal, healthy nails, said treatment being intended to improve their appearance and condition. Such treatment has no therapeutic aim.

*In vitro* and *in vivo* tests detailed below in the description demonstrate the effects of the cosmetic treatment according to the invention.

The *in vitro* tests show that an extract of *Apium graveolens* comprising a mixture of alkyl phthalides improves the differentiation of keratinocytes and stimulates the production of desmogléin-1 (constituent element of desmosomes), which will have a beneficial effect on the growth and strength of the nails and their regeneration in the case of damaged nails.

An *in vivo* test carried out over 4 months confirms these observations by showing that the treatment according to the invention increases the density of the matrix of the nails as well as their rate of growth. Furthermore, the applicant has demonstrated a decrease in the dryness of the nails, which is accompanied by a reduction in ridges.

According to the treatment of the invention the nails become more beautiful and healthy.

"Alkyl-phthalide" are phthalides in which the 6-carbon ring is aromatic and hydro-phthalides in which the 6-carbon ring has only 1 or 2 unsaturated bonds.

An alkyl-phthalide has therefore the following general formula I:

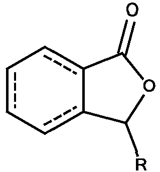

R being an alkyl chain of 1 to 8 carbon atoms, preferably 1 to 4, and preferably 4 (butyl phthalides), linear or branched, that can be substituted by an OH or amine function (secondary or tertiary).

Pure alkyl-phthalides can be obtained by chemical synthesis or by extraction and purification from a plant comprising them.

The present invention requires a plant extract of *Apium graveolens* comprising sedanenolide, sedanolide and 3-n-butylphthalide. These three alkyl-phthalides, shown thereafter, are those comprised in the volatile portion of the seeds of the *Apium graveolens* plant.

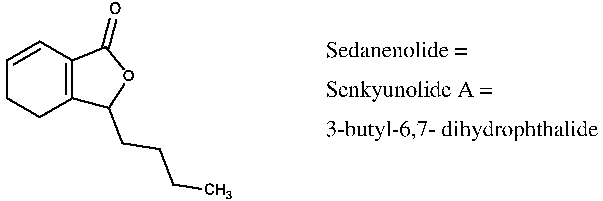

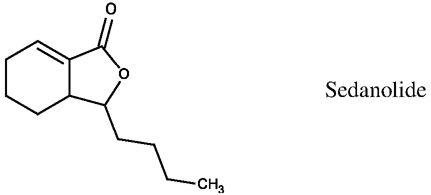

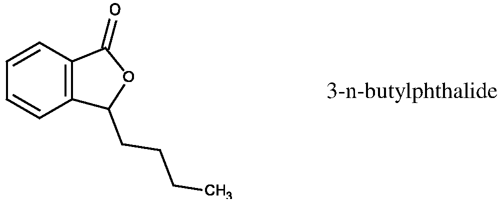

Thus, according to the invention, an extract of *Apium graveolens* seeds comprising sedanenolide, sedanolide and 3-n-butylphthalide is prepared by CO₂ supercritical extraction. The extract comprises alkyl-phthalides as major compounds, namely at least 50%, preferably at least 65%, the remaining compounds mainly consisting of terpenes and traces of fatty acids.

Advantageously, the extract according to the invention comprises among the 3 alkyl-phthalides the sedanenolide as the major compound, preferably comprising at least 50%, and preferably at least 60%, and more preferably at least 70% of sedanenolide based on total phthalides, is used.

The extract is prepared by CO₂ supercritical extraction in a pressure range of 75 to 300 bars (75.10⁵ to 300.10⁵ Pa) and in a temperature range of 30°C to 80°C. Preferably the extraction will be done under 90 bars (90.10⁵ Pa pressure and at 40°C.

Other extraction methods can be used to obtain an extract according to the invention including extraction with a nonpolar solvent such as hexane.

### DETAILED DESCRIPTION

### Preparation of compositions for implementing the present invention

Various types of compositions may be used for a nail treatment, such as:
- A varnish (lacquer or water) that will be applied only on the nail plate;
- A cream/lotion oil/gel that will be applied either on the whole phalanx of the finger or on a specific part, for example at the base of the nail at the level of the cuticle and/or the matrix of the nail;
- A mask, preferably applied to the base of the nail but which could also cover the plate of the nail in the manner of a varnish;
- An adhesive care product that can be peeled after acting;
- A bath;
- A patch; or
- A dissolving lotion.

Each composition for a treatment according to the invention will comprise the plant extract containing said alkyl phthalide mixture as the main active component in a physiologically acceptable medium which will differ according to the type of composition.

"Physiologically acceptable medium" means according to the present invention, without limitation, a base of varnish either solvent or aqueous, an aqueous or hydro-alcoholic solution, a water-in-oil emulsion, an oil-in-water emulsion, a micro-emulsion, an aqueous gel, an anhydrous gel, a serum, a dispersion of vesicles, or a powder.

"Physiologically acceptable" means that the compositions are suitable for topical or transdermal use, in contact with mucous membranes, appendages (nails, hairs), scalp and skin of mammals, particularly human, compositions which may be ingested. or injected into the skin, without risk of toxicity, incompatibility, instability, allergic response, and others. This "physiologically acceptable medium" forms what is commonly called the excipient of the composition.

The alkyl-phthalide plant extract of the invention may be combined with other active ingredients at effective concentrations that can act synergistically or additionally for reinforcing and achieving the desired effects described for the invention, such as the following agents: moisturizing, anti-aging, vitamins, radiation filtering, in particular UVA and/or UVB, stimulating keratinocytes, antioxidants, antiglycation, etc.

The treatment of the invention may be applied in whatever form or carrier known to those skilled in the art, in particular in the form of solution, dispersion, emulsion, paste, or powder, individually or as a premix or in vehicles individually or as a premix in vectors such as macro-, micro- or nano-capsules, macro-, micro- or , nano-spheres, liposomes, oleosomes or chylomicrons, macro-, micro-, or nanoparticles or macro-, micro- or nano-sponges, micro- or nano-emulsions or adsorbed on organic polymer powders, talcs, bentonites, spores or exines, and other inorganic or organic supports.

In general, the alkyl-phthalide plant extract according to the invention may be used in any form, in a form bound to or incorporated in or absorbed in or adsorbed on macro-, micro-, and nanoparticles, or macro-, micro-, and nano-capsules, for the treatment of textiles, natural or synthetic fibres, wools, and any materials that may be intended to come into contact with the skin, handkerchiefs, cloths, gloves, for example, to exert their cosmetic effect via this skin/textile contact and to allow continuous topical delivery.

The CTFA (« International Cosmetic Ingredient Dictionary & Handbook » (16th Ed. 2016) published by « the Personal Care Products council », ex- « the Cosmetic, Toiletry, and Fragrance Association, Inc. », Washington, D.C.), describes a non-limited wide variety of cosmetic and pharmaceutical ingredients commonly used in the skin care industry, which are suitable for use as additional ingredients in the compositions according to the present invention.

Further additional skin care actives that are particularly useful can be found in the commercial literature of Sederma and on the website www.sederma.com.

The following commercial actives can also be mentioned, as examples: betaine, glycerol, Actimoist Bio 2^{™} (Active organics), AquaCacteen^{™} (Mibelle AG Cosmetics), Aquaphyline^{™} (Silab), AquaregulK^{™} (Solabia), Carciline^{™} (Greentech), Codiavelane^{™} (Biotech Marine), Dermaflux^{™} (Arch Chemicals, Inc), Hydra'Flow^{™} (Sochibo), Hydromoist L^{™} (Symrise), RenovHyal^{™} (Soliance), Seamoss^{™} (Biotech Marine), Argireline^{™} (commercial name for the acetyl hexapeptide-3 of Lipotec), spilanthol or an extract of *Acmella oleracea* known under the commercial name Gatuline Expression^{™}, an extract of *Boswellia serrata* known under the commercial name Boswellin^{™}, Deepaline PVB^{™} (Seppic), Syn-AKE^{™} (Pentapharm), Ameliox^{™}, Bioxilift^{™} (Silab), PhytoCellTec^{™}Argan (Mibelle), Papilactyl D^{™} (Silab), Preventhelia^{™} (Lipotec), and from Sederma: Subliskin^{™}, Venuceane^{™}, Moist 24^{™}, Vegesome Moist 24^{™}, Essenskin^{™}, Juvinity^{™}, Revidrat^{™}, Resistem^{™}, Chronodyn^{™}, Kombuchka^{™}, Chromocare^{™}, Calmosensine^{™}, Glycokin factor S^{™}, Biobustyl^{™}, Idealift^{™}, Ceramide 2^{™}, Ceramide A2^{™} et Ceramide HO3^{™}, Legance^{™}, Intenslim^{™}, Prodizia^{™}, Beautifeye^{™}, Pacifeel^{™}, NG-shea butter unsaponifiables (natural grade), Zingerslim^{™}, Meiritage^{™}, Senestem^{™}, Sebuless^{™}, Majestem^{™}, Rubistem^{™}, Citystem^{™}, or mixture thereof.

Among other plant extracts which can be combined with the alkyl phthalide plant extract of the invention, there may more particularly be mentioned extracts of Ivy, in particular English Ivy *(Hedera helix),* of *Bupleurum chinensis,* of *Bupleurum falcatum,* of arnica (*Arnica montana L*), of rosemary (*Rosmarinus officinalis N*), of marigold (*Calendula officinalis*)*,* of sage (*Salvia officinalis L*)*,* of ginseng (*Panax ginseng*)*,* of ginko biloba, of St.-John's-Wort (*Hyperycum perforatum*)*,* of butcher's-broom (*Ruscus aculeatus L*)*,* of European meadowsweet (*Filipendula ulmaria L*)*,* of big- flowered Jarva tea (*Orthosiphon stamincus benth*)*,* of artichoke (*Cynara scolymus*)*,* of algae (*Fucus vesiculosus*)*,* of birch (*Betula alba*)*,* of green tea, of cola nuts (*Cola nipida*)*,* of horse-chestnut, of bamboo, of *Centella asiatica,* of heather, of fucus, of willow, of mouse-ear, of escine, of cangzhu, of *chrysanthellum indicum,* of the plants of the *Armeniacea* genus, *Atractylodis platicodon, Sinnomenum, Pharbitidis, Flemingia,* of *Coleus* such as *C*. *Forskohlii, C. blumei, C. esquirolii, C. scutellaroides, C. xanthantus* and *C*. *Barbatus,* such as the extract of root of *Coleus barbatus,* extracts of *Ballote,* of *Guioa,* of *Davallia,* of *Terminalia,* of *Barringtonia,* of *Trema,* of *antirobia, cecropia, argania, dioscoreae* such as *Dioscorea opposita* or Mexican, extracts of *Ammi visnaga,* of *Siegesbeckia,* in particular *Siegesbeckia orientalis,* vegetable extracts of the family of *Ericaceae,* in particular bilberry extracts (*Vaccinium angustifollium*) or *Arctostaphylos uva ursi, aloe vera,* plant containing sterols (e.g., phytosterol), Manjistha (extracted from plants of the genus *Rubia,* particularly *Rubia cordifolia*), and Guggal (extracted from plants of the genus *Commiphora,* particularly *Commiphora mukul*), kola extract, chamomile, red clover extract, Piper methysticum extract (Kava Kava^{™} from Sederma), *Bacopa monieri* extract (Bacocalmine^{™} from Sederma) and sea whip extract, extracts of *Glycyrrhiza glabra,* of mulberry, of *melaleuca* (tea tree), of *Larrea divaricata,* of *Rabdosia rubescens,* of *Euglena gracilis,* of *Fibraurea recisa Hirudinea,* of *Chaparral Sorghum,* of sun flower extract, of *Enantia chlorantha,* of Mitracarpe of *Spermacocea* genus, of *Buchu barosma,* of *Lawsonia inermis L.,* of *Adiantium capillus-veneris L.,* of *Chelidonium majus,* of *Luffa cylindrica,* of Japanese Mandarin (*Citrus reticulata Blanco* var. *unshiu*)*,* of *Camelia sinensis,* of *Imperata cylindrica,* of *Glaucium Flavum,* of *Cupressus sempervirens,* of *Polygonatum multiflorum,* of *loveyly hemsleya,* of *Sambucus nigra,* of *Phaseolus lunatus,* of *Centaurium,* of *Macrocystis pyrifera,* of *Turnera diffusa,* of *Anemarrhena asphodeloides,* of *Portulaca pilosa,* of *Humulus lupulus,* of *Coffea arabica,* of *Ilex paraguariensis,* or of *Globularia cordifolia,* of *Albizzia julibrissin,* of *Oxydendron arboretum,* of *Zingimber zerumbet smith,* of *Astragalus membranaceus,* of *Atractylodes macrocephalae,* of *Plantago lanceolata,* of *Leontopodium alpinum,* of *Mirabilis jalapa,* of *Marrubium vulgare,* or of orchids.

The compositions of the present invention may include one or more peptides, including, without limitation, di-, tri-, tetra-, penta-and hexapeptides and their derivatives. According to a particular embodiment, the concentration of the additional peptide, in the composition, ranges from 1x10⁻⁷% and 20%, preferably from 1x10⁻⁶% and 10%, preferably between 1x10⁻⁵% and 5% by weight.

According to the present invention, the term "peptide" refers to peptides containing 10 amino acids or less, their derivatives, isomers and complexes with other species such as a metal ion (e.g. copper, zinc, manganese, magnesium, and others). The term "peptides" refers to both natural peptides and synthetic peptides. It also refers to compositions that contain peptides and which are found in nature, and/or are commercially available.

Suitable dipeptides for use herein include but are not limited to Carnosine (βAH), YR, VW, NF, DF, KT, KC, CK, KP, KK, TT, PA, PM or PP.

Suitable tripeptides for use herein include, but are not limited to RKR, HGG, GKH, GGH, GHG, KFK, KAvaK, KβAK, KAbuK, KAcaK, KPK, KMOK, KMO₂K (MO₂ being a di-oxygenated sulfoxide methionine), KVK, PPL, PPR, SPR, QPA, LPA or SPA.

Suitable tetrapeptides for use herein include but are not limited to RSRK (SEQ ID NO: 1), GQPR (SEQ ID NO: 2), KTFK (SEQ ID NO: 3), KTAK (SEQ ID NO: 4), KAYK (SEQ ID NO: 5) or KFYK (SEQ ID NO: 6).

Suitable pentapeptides include, but are not limited to KTTKS (SEQ ID NO: 7). Suitable hexapeptides include but are not limited to GKTTKS (SEQ ID NO: 8) and VGVAPG (SEQ ID NO: 9).

Other suitable peptides for use herein include, but are not limited to: lipophilic derivatives of peptides, preferably palmitoyl (Pal) derivatives, and metal complexes as aforementioned (e.g. copper complex of the tripeptide HGG). Preferred dipeptide include for example N-Palmitoyl-β-Ala-His, N-Acetyl-Tyr-Arg-hexadecylester (Calmosensine^{™}, Idealift^{™} from Sederma), Pal-RT or Pal-KT (Sederma). Preferred tripeptide derivatives include for example Pal-GKH and Pal-GHK (from Sederma), the copper derivative of HGG (Lamin^{™} from Sigma), Lipospondin (N-Elaidoyl-KFK) and its analogs of conservative substitution, N-Acetyl-RKR-NH₂ (Peptide CK+), N-Biot-GHK (from Sederma), Pal-KAvaK, Pal-KβAlaK, Pal-KAbuK, Pal-KAcaK, or Pal-KMO₂K (Matrixyl^{®}synthe'6^{®} from Sederma), PalKVK (Syn-Coll^{™} of DSM), and derivatives thereof.

Here can also be cited the anti-aging tripeptides of general formula X-Pro*-Pro*-Xaa-Y disclosed in WO2015181688 with Xaa selected from Leu, Arg, Lys, Ala, Ser, and Asp; at the N terminal end, X selected from H, -CO-R₁ and -SO₂-R₁ and at the C terminal end Y selected from OH, OR₁, NH₂, NHR₁ or NR₁R₂; R₁ and R₂ being, independently from each other, selected from an alkyl, aryl, aralkyl, alkylaryl, alkoxy et aryloxy group, that can be linear, branched, cyclic polycyclic, saturated, unsaturated, hydroxylated, carbonylated, phosphorylated and/or sulfured, said group having or not an O, S and/or N heteroatom in its skeleton and Pro* corresponding to a Proline, analog or derivative thereof; comprising for example Myr-PPL-OH and Myr-PPR-OH.

Here can further be cited also the propigmenting and/or pro-mec dipeptides and tripeptides of general formula X-(Xaa₁)n-Pro*-Xaa₂-Y disclosed in WO2014/080376, with n=0, 1 or 2, Xaa₁ an hydrophobic aminoacid selected from Ala, Val, Met, Leu, Iso, Phe, Pro, and analogs and derivatives thereof; or a polar aminoacid selected from Ser, Thr, Tyr, Asp, Glu and analogs and derivatives thereof; and when n=2 the two aminoacids Xaa₁ being the same or different; Xaa₂ being an hydrophobic aminoacid selected from Ala, Val, Met, Leu, Iso, Phe, and analogs and derivatives thereof, or a basic aminoacid selected from Arg, Lys, His, and analogs and derivatives thereof; at the N terminal end X being selected from H, -CO-R₁ and -SO₂-R₁; at the C terminal end Y being selected from OH, OR₁, NH₂, NHR₁ or NR₁R₂; R₁; and R₂ being, independently from each other, selected from an alkyl, aryl, aralkyl, alkylaryl, alkoxy et aryloxy group, that can be linear, branched, cyclic polycyclic, saturated, unsaturated, hydroxylated, carbonylated, phosphorylated and/or sulfured, said group having or not an O, S and/or N heteroatom in its skeleton and Pro* corresponding to a Proline, analog or derivative thereof; comprising for example the following peptides Pal-SPR-OH, Pal-PPR-OH, Pal-QPA-OH, Pal-LPAOH, Myr-SPA-OH, Pal-PM-OH, Pal-PA-OH and Pal-PP-OH.

Suitable tetrapeptide derivatives for use according to the present invention include, but are not limited to, Pal-GQPR (SEQ ID NO: 10) (from Sederma) and Pal-KTFK (SEQ ID NO: 11) or Ela-KTFK (SEQ ID NO: 12), Ela-KTAK (SEQ ID NO: 13), Ela-KAYK (SEQ ID NO: 14) or Ela-KFYK (SEQ ID NO: 15). Suitable pentapeptide derivatives for use herein include, but are not limited to, Pal-KTTKS (SEQ ID NO: 16) (available as Matrixyl^{®} from Sederma), Pal-YGGFXaa (SEQ ID NO: 17) with Xaa being Leu or Pro, or mixtures thereof. Suitable hexapeptide derivatives for use herein include, but are not limited to, Pal-VGVAPG (SEQ ID NO: 18), Pal-GKTTKS (SEQ ID NO: 19), Pal-HLDIIXaa with Xaa being Trp, Phe, Tyr, Tic, 7-hydroxy-Tic ou Tpi (SEQ ID NO: 20) and derivatives thereof. The mixture of Pal-GHK and Pal-GQPR (SEQ ID NO: 10) (Matrixyl^{®} 3000, Sederma) can also be mentioned. The preferred compositions commercially available containing a tripeptide or a derivative include Biopeptide-CL^{™}, Maxilip^{™}, Biobustyl^{™}, Procapil^{™} and Matrixyl^{®}synthe'6^{®} of Sederma. The compositions commercially available preferred sources of tetrapeptides include Rigin^{™}, Eyeliss^{™}, Matrixyl^{®} Reloaded and Matrixyl 3000^{®} which contain between 50 and 500 ppm of Pal-GQPR (SEQ ID NO: 10) and an excipient, proposed by Sederma.

The following marketed peptides can be mentioned as well as additional active ingredients:
- Vialox^{™} (INCI name = Pentapeptide-3 (synthetic peptide comprising alanine, arginine, isoleucine, glycine and proline)), Syn-ake^{™} (β-Ala-Pro-Dab-NH-Bzl) or Syn-Coll^{™} (Pal-Lys-Val-Lys-OH) marketed by Pentapharm;
- Argireline^{™} (Ac-Glu-Glu-Met-Gln-Arg-Arg-NH₂ (INCI name = Acetyl hexapeptide-3) (SEQ ID NO: 21), Leuphasyl^{™} (Tyr-D-Ala-Gly-Phe-Leu) (SEQ ID NO: 22), Aldenine^{™} (Gly-His-Lys), Trylagen^{™} (INCI name = Pseudoalteromonas Ferment Extract, Hydro lyzed Wheat Protein, Hydro lyzed Soy Protein, Tripeptide-10 Citrulline (reaction product of Citrulline and Tripeptide-10 (synthetic peptide constituted of aspartic acid, isoleucine and lysine)), Tripeptide-1), Eyeseryl^{™} (Ac-β-Ala-His-Ser-His)(SEQ ID NO: 23), Serilesine^{™} (Ser-Ile-Lys-Val-Ala-Val) (SEQ ID NO 24) or Decorinyl^{™} (INCI name: Tripeptide-10 Citrulline = reaction product of Citrulline and Tripeptide-10 (synthetic peptide constituted of aspartic acid, isoleucine and lysine) marketed by Lipotec;
- Collaxyl^{™} (Gly-Pro-Gln-Gly-Pro-Gln (SEQ ID NO 25)) or Quintescine^{™} (Cys-Gly) marketed by Vincience;
- Cytokinol^{™}LS (casein hydrolysate) marketed by Les Laboratoires Serobiologiques/Cognis;
- Kollaren^{™} (Gly-His-Lys), IP2000^{™} (Pal-Val-Tyr-Val) or Meliprene^{™} (INCI name = Monofluoroheptapeptide-1: reaction product of acetic acide and a synthetic peptide comprising arginine, glycine, glutamic acid, histidine, norleucine, p-fluorophenylalanine and tryptophan) marketed by l'lnstitut Européen de Biologie Cellulaire;
- Neutrazen^{™} (Pal-His-D-Phe-Arg-NH₂) marketed by Innovations; or
- BONT-L-Peptide^{™} (INCI name = Palmitoyl Hexapeptide-19: reaction product of palmitic acid and Hexapeptide-19 (synthetic peptide constituted of asparagine, aspartic acid, lysine and methionine), Timp-Peptide^{™} (INCI name = Acetyl Hexapeptide-20: reaction product obtained by acetylation of Hexapeptide-20 (synthetic peptide constituted of alanine, glycine, lysine, valine and proline) or ECM Moduline^{™} (INCI name = Palmitoyl Tripeptide-28: reaction product of palmitic acid and Tripeptide-28 (synthetic peptide constituted of arginine, lysine and phenylalanine) marketed by lnfinitec Activos.

More specifically, according to the invention the alkyl-phthalide plant extract may be combined with at least one of compounds selected from compounds of the vitamin B3, compounds such as niacinamide or tocopherol, retinoid compounds such as retinol, hexamidine, α-lipoic acid, resveratrol or DHEA, hyaluronic acid, peptides, in particular N-acetyl-Tyr-Arg-O-hexadecyl ester, Pal-VGVAPG (SEQ ID NO: 18), Pal-KTTKS (SEQ ID NO: 16), Pal-GHK, Pal-KMO₂K and Pal-GQPR (SEQ ID NO: 10), which are widely used active ingredients in topical cosmetic or dermopharmaceutical compositions.

More specifically also, according to the invention, the alkyl phthalide plant extract can be combined with at least one of the following compounds known for their efficacy for nail care: amino acids (including arginine, lysine, cysteine and methionine); Proteins (especially wheat); Trace elements (especially Cu, Zn); Silicon; Vegetable oils such as Argan, Siberian pine, Date palm, Coconut; Tea, Melaleucale oils, Shea butter (especially unsaponifiables); Beeswax, ceramides, marine crystals, vitamins B, D and E, keratin, isolated keratin, D-pantenol, Ginkgo biloba, etc., optionally encapsulated, for example, in the form of nanoparticles to promote their formulation.

The use according to the invention improves the appearance and general condition of the nails. The use according to the invention allows in particular increasing the rate of nail growth, increasing the thickness of the nail plate, increasing the density of the nail matrix and/or decreasing the water loss of the nails.

"Topical treatment" or "topical use" means according to the invention, an application that is intended to act where it is applied.

A composition according to the invention may be applied locally to targeted areas, for example using a cannula type of applicator.

The "effective" amount depends on various factors, such as the age, the severity of the disorder and the administration mode, etc. An effective amount means a non-toxic amount enough to achieve the desired effect.

All percentages and ratios used herein are by weight of the total composition and all measurements are made at 25°C unless it is otherwise specified.

For example, the European Cosmetics Directive has set a standard amount for a varnish of 0.3g/application (for whole nails) and a mean amount by day of 0.04 to 0.05g for a mean frequency of use of 1,17 times/week.

According to other specific features, the cosmetic treatment method according to the invention can be combined with one or more other treatment methods targeting the skin such as lumino-therapy, heat or aromatherapy treatments.

According to the invention, devices with several compartments or kits may be proposed to apply the method described above which may include for example and non-restrictively, a first compartment containing a composition comprising the alkyl-phthalide plant extract according to the invention, and in a second compartment another active ingredient and/or excipient, the compositions contained in the said first and second compartments in this case being considered to be a combination composition for simultaneous, separate or stepwise use in time, particularly in one of the treatment methods recited above.

### A) Example of preparation of an Apium graveolens seeds extract that can be used in the context of the invention

The *Apium graveolens* seeds are ground to a particle size of powder around 800µm. This powder is then extracted with supercritical CO₂ under 90 bars pressure (90.10⁵ Pa) and at 40°C. The residual water that may be present in the final extract is then removed if necessary (for example by decantation, vacuum evaporation, and lyophilization). The extraction yield is around 2.5%. The extract is in the form of an oily liquid, clear to slightly opalescent, colorless to pale yellow.

The resulting extract is passed on Ultra High Performance Liquid Chromatography (UHPLC) on a C18 column with as mobile phase a water/acetonitrile gradient, to dose the various phthalides.

This analysis confirms the presence of 71% of total phthalides in the extract comprising the 3-alkyl phthalides: 10% of 3-n-butylphthalide, 30% of sedanolide and 60% of sedanenolide by weight relative to the total phthalides. The extract can be used pure or diluted.

### B) Formulation of an active ingredient that can be used in the context of the invention

The active ingredient is a composition comprising the extract of *Apium graveolens* seeds obtained according to A) above dissolved in a matrix forming a physiologically acceptable medium. This active ingredient is particularly intended for the cosmetics industry for the preparation of cosmetics, creams, gels, etc. (See galenic examples at point D) below).

An extract obtained according to A) above can be diluted in any physiologically acceptable fatty excipient to reach at the end a concentration of 500ppm of alkyl-phthalides. Esterified oil is preferably used of Caprylic/Capric Triglyceride type for this dilution.

For example, and for the description of the *in vivo* tests and the galenic of point D) below, it is this dilution that has been preferably used. This constitutes the active ingredient that will be used itself preferably at between 1 and 5% in a cosmetic composition that can be applied on the nails.

### C) In vitro test results

*In vitro* tests were carried out from a crude extract of seed oil of *Apium graveolens* manufactured according to the production method described in A) above.

### 1. Mechanism of action

### a. DNA array study

Normal human keratinocytes (KHN) at confluence were contacted with the extract. At the end of the incubations, the cell layers, once rinsed, were ground and the mRNAs were extracted in a standardized manner. An analysis of the transcripts was then carried out using DNA chips according to the DNA-Array technique. An analysis of the data obtained has confirmed that several genes involved in the epidermal differentiation and reinforcement were overexpressed early after 6 hours in the presence of the extract.

Overexpression was observed for the genes encoding the involucrin (x2.02), filaggrins 1 & 2 (x2.59 and 24.9), loricrin (x4.93), cornulin (x2.38) keratin maturation and terminal differentiation Keratin-1 and - 10 (x4.42 and 2.57). In addition, other genes related to hard keratins are overexpressed in parallel: Keratin-81 (x1.51, only at 24h), Keratin-85 and Keratin-86 (x1.75 and 1.53).

An induction of the expression of the trichohyalin gene (x5.53), cement of the keratins of the nail was also observed. Furthermore, two genes encoding WNT ligands were induced very noticeably: WNT10A (x1.93 and 2.27 after 6 and 24h) and WNT5A (x3.24 after 24h). The WNT pathway is known to play a role in the maintenance of adult tissues but also in the growth of the integuments including the nails.

The analysis of these results shows that the extract can not only improve the terminal differentiation of epidermal keratinocytes but also strengthen and improve the resistance of the nails.

### b. Determination of WNT production by Elisa technique

As it was not possible to obtain nail KHNs, normal human keratinocytes were used. These cells at subconfluence were contacted with the extract. At the end of this incubation, the carpets of cells were rinsed and ground to recover the total proteins. After treatment, these preparations were centrifuged and an amount of WNT10A was assayed on the supernatants. A total protein assay was performed in parallel to normalize the results.

**Table 1: Variation of the production of WTN by keratinocytes in contact with the extract of Apium graveolens**

| | **WNT 10A (pg/1mg proteins)** | **Variation** |
|---|---|---|
| **Control** | 389.1 ± 33.6 | Reference |
| Extract according to the invention | 580.3 ± 62.4 | **+49%** ; ***p<0.01*** |

### c. Determination of DKK1 by Elisa technique

DKK1 is the natural inhibitor of the WNT/β-catenin pathway.

Normal human keratinocytes with sub-confluence were contacted with the extract of the invention. At the end of this incubation, the carpets of cells were rinsed and ground to recover the total proteins. After treatment, these preparations were centrifuged and an amount of DKK1 was assayed on the supernatants. A total protein assay was performed in parallel to normalize the results.

**Table 2: Variation of the production of DKK1 by keratinocytes in contact with the extract of Apium graveolens**

| | **WNT 10A (pg/1mg proteins)** | **Variation** |
|---|---|---|
| **Control** | 7235.9 ± 275.3 | Reference |
| Extract according to the invention | 3959.6 ± 158.0 | **-45% ; *p<0.01*** |

These results show that in parallel with the induction of an activator of the WNT/β-catenin pathway, the extract of the invention has the capacity to repress the synthesis of its inhibitor, the DKK1 protein.

In conclusion, the extract according to the invention is a good inducer of the WNT/β-catenin pathway known to promote the formation, growth and regeneration of the nail.

### 2. Improvement of keratinocyte differentiation and cornification proteins

The tests were performed on skin keratinocytes, close to nail keratinocytes.

### a. Observation in fresh condition:

Normal human keratinocytes almost at confluence were placed in contact with the *Apium graveolens* extract (8 ppm) in a suitable culture medium to study their differentiation under the microscope by following the appearance of the carpets compared to a case control. The aspect of differentiation is assessed visually at 3 days. The *Apium graveolens* extract shows a clear acceleration of differentiation with a stimulation of the formation of typical structures of a keratinocyte in the way of differentiating (presence of branched structures characteristic of the rigid proteolipid matrix forming a refractive network). This mechanism is similar to that found in the nail matrix during its formation. The differentiated profile is not visible (or much less) on the control cellular carpets.

### b. Proteins of cornification: involucrin, loricrin, LCE3B, SPRR

Principle: The same cellular mats as those studied above in the fresh state were fixed and immunolabelled in order to visualize the synthesis of various epidermal differentiation markers: involucrin, loricrin, LCE3B and SPRR. 5 photos were made on each of the 3 replicas. Quantification of the markings was carried out by image analysis. A counterstaining of the cell nuclei is used to estimate the number of cells and thus to standardize the data. Synthesis of LCE3B and SPRR markers was assessed by ELISA on the same cells

### • Involucrin

**Table 3: Variation of the production of Involucrin by keratinocytes in contact with the extract of Apium graveolens (15 photos/case)**

| | | **Involucrin (AFU*/10⁶cell.)** | **Variation** |
|---|---|---|---|
| **Control** | | 7.5 +/- 9.4 | Reference |
| **Extract according to the invention** | **8 ppm** | 206 +/- 94 | **x 27;** *p<0.01* |
| | **16 ppm** | 1134 +/- 291 | **x 151;** *p<0.01* |
| | **24 ppm** | 1014 +/- 74 | **x 135;** *p<0.01* |

### • Loricrin

**Table 4: Variation of the production of Loricrin by keratinocytes in contact with the extract of Apium graveolens**

| | | **Loricrin (AFU*/10⁶cell.)** | **Variation** |
|---|---|---|---|
| **Control** | | 39 +/- 28 | Reference |
| **Extract according to the invention** | **8 ppm** | 149 +/- 76 | **x 3.8;** *p<0.01* |
| | **16 ppm** | 491 +/- 369 | **x 12.6;** *p<0.01* |

### • LCE3B and SPRR2B

Late corneal envelope proteins (LCE) are expressed late in the process of differentiation of keratinocytes and facilitate the formation of the horny envelope. There are three groups of LCE proteins: LCE1, LCE2 and LCE3 encoded by 17 genes. It was disclosed that members of the LCE3 group participated in repairing the skin barrier while other LCE proteins played a role in the normal barrier function.

**Table 5: Variation of the production of LCE3B by keratinocytes in contact with the extract of Apium graveolens**

| | | **LCE3B (pg/ml/10⁶ cells)** | **Variation** |
|---|---|---|---|
| **Control** | | 498 ± 66 | Reference |
| **Extract according to the invention** | **8 ppm** | 702 ± 76 | **+41%; *p<0.05*** |
| | **16 ppm** | 751 ± 44 | **+51%; *p<0.01*** |

At the same time, production of the SPRR2B protein (SPRR marker) was increased in the keratinocyte culture by 19% and 103% (p<0.01) in contact with 8ppm and 16ppm of the extract, respectively, compared to the control.

The extract of the invention stimulates in a dose-dependent manner the synthesis of the involucrin, loricrin, LCE3B and SPRR proteins and thus participates in the proper architecture of the nail plate through a good quality cornification. All these results concur to demonstrate the role of the extract of the invention in restoring and maintaining the nail structure.

### c. Ex vivo testing of keratin production

Nails were sampled from the volunteers who were involved in the clinical testing (see below point E). The nails were cut with nail clippers, then sliced using a cryomicrotome and finally extracted. Dot-blot testing was performed on the insoluble fraction for target proteins. Immunolabelling was also performed on histological sections of these samples.

### • K6 and K17 by Dot-Blot

The Dot-Blot technique is used for measuring the extracted and deposited proteins on a membrane. The protein is targeted by a specific antibody and revealed by chemiluminescence. The collected nails were crushed after freezing and the protein fraction was recovered using an urea buffer. The insoluble fraction thus obtained contains most of the proteins extracted from these samples. A constant amount of proteins was deposited on the Dot-Blot membranes and, after drying, the targeted proteins sought as indicated above, the labellings were revealed by a CCD image camera. The obtained spots were digitized and the results were standardized using spots of a control protein and then compared to T0 controls.

Two proteins were observed: keratin-6 and -17.

A study of the variances and a statistical analysis by Student's t test were carried out to judge the significance of the results.

**Table 6: Variation of K6 and K17 production in nails after a 4-month treatment with the extract of Apium graveolens**

| **Dot-Blot** | **K6 (ALU*)** | | **K17 (ALU*)** | |
|---|---|---|---|---|
| n=16 | **TO** | **T4 months** | **T0** | **T4 months** |
| Mean | 0.76 | 1.06 | 0.55 | 0.83 |
| Standard deviation | 0.36 | 1.00 | 0.36 | 0.77 |
| Variation *vs* T0 | - | **40.8%** | - | **52.7%** |
| Significance | - | *p<0.01* | - | *p<0.01* |

The results show that the production of keratin-6 and keratin-17 were highly stimulated by the treatment according to the invention. This result was confirmed in vitro on keratinocytes.

### • K16 and K31 by immunochemistry

Some of the nails recovered were finely sliced with the cryomicrotome so as to have cross sections of each nail. These sections were assembled and marked with specific antibodies for keratin-16 and keratin-31. These specific stainings made it possible to quantify these proteins and the images obtained were analyzed.

**Table 7: Variation of K16 and K31 production in nails after a 4-month treatment with the extract of Apium graveolens**

| **Immunofluorescence** | **K16 (ALU*)** | | **K31 (ALU*)** | |
|---|---|---|---|---|
| n=75 | **T0** | **T4 months** | **T0** | **T4 months** |
| Mean | 0.496 | 0.958 | 0.066 | 0.076 |
| Standard deviation | 0.317 | 0.636 | 0.031 | 0.044 |
| Variation *vs* T0 | - | **93.0%** | - | **13.8%** |
| Significance | - | *p<0.01* | - | *p<0.01* |

The results on hard and soft keratins confirm the effects on the nail building and structuring.

### 3. Improvement of barrier function and cell cohesion

### a. Neutral lipids, ceramide 2 and filaggrin:

### • Neutral lipids

A culture normal human keratinocytes was labelled using a stain specific to neutral lipids (cholesterol and derivatives, triglycerides, fatty acids). After photos were taken, image analysis was used to estimate the quantity of neutral lipids. The quantity of cells was estimated using a nuclei counter stain.

**Table 8: Variation of the production of neutral lipids by keratinocytes in contact with the extract of Apium graveolens**

| | | **Lipids (AFU*/10⁶cell.)** | **Variation** |
|---|---|---|---|
| **Control** | | 22 +/- 11 | Reference |
| **Extract according to the invention** | **8 ppm** | 132 +/- 85 | **x6; *p<0.01*** |
| | **16 ppm** | 233 +/- 68 | **x10.7; *p<0.01*** |

| | | | |
|---|---|---|---|
| ** AFU: Arbitrary Fluorescence Unit; no cytotoxic effect noted* | | | |

### • Ceramides on keratinocytes culture and explant

A culture similar to that above was labelled with a ceramide 2-specific antibody. After photos were taken, image analysis was used to estimate the quantity. The quantity of cells was estimated using a nuclei counter stain. Four skin explants were immunolabelled to reveal the changes in ceramide 2 content. After photos were taken (n=48), image analysis was used to estimate the quantity.

**Table 9: Variation of the production of ceramide 2 by keratinocytes and explant in contact with the extract of Apium graveolens**

| | **Keratinocytes** | | **Explants** | |
|---|---|---|---|---|
| | **Ceramide 2*** | **Variation** | **Ceramide 2*** | **Variation** |
| **Control/Placebo** | 456 ± 471 | Reference | 18.9 ± 7.2 | Reference |
| **Extract according to the invention**** | 944 ± 364 | **+107%; p<0.01** | 26.4 ± 11.2 | **+39.4%; p<0.01** |

| | | | | |
|---|---|---|---|---|
| ** AFU: Arbitrary Fluorescence Unit; no cytotoxic effect noted* *** 8 and 16ppm in non-rinsed lotion for explants.* | | | | |

### • Filaggrin

**Table 10: Variation of the production of filaggrin by keratinocytes in contact with the extract of Apium graveolens (15photos/case)**

| | | **Filaggrin (AFU*/10⁶cell.)** | **Variation** |
|---|---|---|---|
| **Control** | | 182 +/- 150 | Reference |
| **Extrait according to the invention** | **8 ppm** | 460 +/- 263 | **+153%;** *p<0.01* |
| | **16 ppm** | 613 +/- 182 | **+237%;** *p<0.01* |
| | **24 ppm** | 347 +/- 138 | **+91%;** *p<0.01* |

| | | | |
|---|---|---|---|
| ** AFU: Arbitrary Fluorescence Unit; no cytotoxic effect noted.* | | | |

### b. Reinforcement of cell cohesion of the nail

The desmosomes are mechanical junctions allowing the cells to adhere to one another. They contribute to the cohesion and therefore to the solidity of a tissue by the reinforcement of the intercellular junctions. These desmosomes have been described in the nail. Moreover, a deficiency in certain proteins of the desmosome, such as, for example, desmoglein-1, is at the origin of pathologies in the level of the nail. The effect of the extract of *Apium graveolens* according to the invention has therefore been studied on the production level of desmoglein-1 in scalp explants (closest approach, it is not possible to obtain nail explants).

**Principle:** Scalp explants (diameter: 5 mm) are cultured in media containing 24 ppm of the extract of *Apium graveolens* according to the invention or its placebo. At the end of this contact (3 days), the skins are rinsed, frozen. Then 7µm cryosections are made using the microtome.

The immuno-marked sections are observed under a fluorescence microscope and the intensity of fluorescence is quantified by Image J.

**Table 11: Variation of the production by scalp explants in contact with the extract of Apium graveolens according to the invention (12 photos/case)**

| | | **Desmogleine-1 (AFU)** | **Variation** (%) |
|---|---|---|---|
| **Control** | | 25.2 +/- 5.7 | Reference |
| **Extract according to the invention** | **24 ppm** | 29.1 +/- 6.9 | **+15.4%** ; *p<0.01* |

24ppm of the *Apium graveolens* seed extract of the invention induce a significant increase in the synthesis of desmoglein-1 on scalp explant. The product will act favorably on the cohesion of the cells of the nail.

The same cells as those described previously were also set and immunolabelled in order to observe synthesis of the various intercellular junction specific molecules: claudin, occludin, ZO-1, corneodesmosin. For each target molecule, the labelling reveals an improvement of the architecture. In the case of the control, the labelling is diffuse in the cytoplasm. With the extract of the invention, the junction molecules rearrange in the cell membrane.

Adequate intercellular cement lipidic composition and optimal cell cohesion ensure tissue integrity and proper barrier function which significantly influences water loss.

### D) Galenic

Various cosmetic formulations are described below. Additional active ingredients, coming when appropriate in support and/or in addition to the activity of the active ingredient according to the invention can be added in the correct formulation part according to their hydrophobic or hydrophilic nature. These ingredients can be of any category according to their(s) function(s), the desired end.

**Active ingredient according to the invention** used in the galenic formulations given below: CO₂ supercritical extract of *Apium graveolens* seeds included in an ester oil of Caprylic/Capric Triglyceride type so as to be at the end at 500 ppm of total alkyl phthalides.

This ingredient is preconized between 0.1 and 10%, preferably between 1 and 5%, more preferably between 2 and 3%.
**1) Lacquer varnish**

| **Ingredient** - **INCI name** | (%) | **Role** |
|---|---|---|
| Ethyl Acetate | 25-50 | solvent |
| Butyl Acetate | 25-50 | solvent |
| Nitrocellulose | 10-25 | lacquer |
| Adipic Acid / Neopentyl Glycol / Trimellitic Anhydride Copolymer | 5-10 | plastifier |
| Isopropyl Alcohol | 5-10 | solvent |
| Acetyl Tributyl Citrate | 5-10 | plastifier |
| Ingredient actif pour l'invention | 2 | Cosmetic active |
| Isosorbide Dicaprylate / Caprate | 0.1 - 1 | Plastifier |
| N-Butyl Alcohol | 0.1 - 1 | Solvent |
| Etocrylene | 0.1 - 1 | Rheology agent |
| Trimethylpentanediyl Dibenzoate | 0-0.1 | Plastifier |

2) **Detoxifying bath** (clear concentrated lotion to be diluted to 50% by adding it to water, pH = 5.50 +/- 0.20)

| **Product** | **%** | **INCI name** | **Role** |
|---|---|---|---|
| **Part A** | | | |
| H₂O | Qsp100 | Water | - |
| Potassium sorbate | qs | Potassium Sorbate | Preservative |
| Citric acid | qs | Sodium Citrate | Buffer |
| Trisodic citrate | qs | Potassium Sorbate | Buffer |
| Synperonic PE/L 64 | 2.00 - 0.50 | Poloxamer 184 | Solubiliser |

| **Part B** | | | |
|---|---|---|---|
| Croduret 54-SO-(MV) | 6.00 - 4.00 | PEG-54 Hydrogenated Castor Oil | Solubiliser |
| Cromollient SCE - LQ-(MH) | 2.00 - 1.00 | Di-PPG-2 Myreth-10 Adipate | Fatty solubiliser |
| Active ingredient of the invention | 2.00 | - | Cosmetic active |

| **Part C** | | | |
|---|---|---|---|
| Glycerin | 10.00 | Glycerin | Humectant |
| Phenoxyethanol | qs | Phenoxyethanol | Preservative |

| **Part D** | | | |
|---|---|---|---|
| Tween 20-LQ-(MV) | 2.00 - 1.00 | Polysorbate 20 | Solubilizer |

| **Part E** | | | |
|---|---|---|---|
| Coralline^{™} | 1.00 | - | Cosmetic active |

| **Part F** | | | |
|---|---|---|---|
| Citystem^{™} | 2.00 | - | Cosmetic active |

| **Part G** | | | |
|---|---|---|---|
| Keratoline^{™} | 0.50 | - | Cosmetic active |

- **Keratoline**^{™}**:** active ingredient marketed by Sederma, based on a protease obtained by fermentation of *Bacillus subtilis;* cell renewal is stimulated thanks to its enzymatic action.
- **CITYSTEM**^{™}**:** active ingredient marketed by Sederma based on plant cells obtained *in vitro* from *Marrubium vulgare* with a high concentration of Forsythoside B; used against the attacks of pollution: makes the skin soft and smooth, refines the skin texture, reduces the visibility of comedones, leaving the skin radiant and purified.
- **Coralline**^{™} : active ingredient marketed by Crodarom, extracted from the red alga Corallina Officinalis (vegetable coral), used for its antioxidant, antibacterial, moisturizing and regenerating properties thanks to the presence of minerals, polysaccharides and phenolic compounds.

**3) Balm patch**

| **Product** | **%** | **INCI name** | **Role** |
|---|---|---|---|
| **Part A** | | | |
| H₂O | Qsp100 | Water | Excipient |
| Viscoptima LV-LQ-(RB) | 3.00 | Sodium Polyacrylate & Isotridecyl Isonanoate & Trideceth-6 | Rheology agent |
| Sorbate de Potassium | Qs | Potassium Sorbate | Preservative |

| **Part B** | | | |
|---|---|---|---|
| Glycerin | 3.00 - 7.00 | Glycerin | Humectant |
| Phenoxyethanol | Qs | Phenoxyethanol | Preservative |

| **Part C** | | | |
|---|---|---|---|
| Steartic acid | 8.00 - 6.00 | Stearic Acid | Co-surfactant |
| Syncrowax HRC-PA-(RB) | 16.00 -12.00 | Tribehenin | Cire |
| Ceramide 2 | 0.10 | Ceramide NG | Cosmetic active |
| Oleocraft LP-20-PA-(MV) | 16.00 - 20.00 | Polyamide-8 | Thickener |
| Crodamol STS-LQ-(MH) | 1.00 - 2.00 | PPG-3 Benzyl Ether Myristate | Dry ester |
| Crodamol AB-LQ-(RB) | 6.00-9.00 | C12-15 Alkyl Benzoate | Dry ester |

| **Part D** | | | |
|---|---|---|---|
| Invention ingredient | 2.00 | | Cosmetic active |

| **Part E** | | | |
|---|---|---|---|
| White Tea^{™} | 0.50 - 2.00 | - | Cosmetic active |

| **Part G** | | | |
|---|---|---|---|
| H₂O | Qs | Water | |
| NaOH 30 % | Qs | Sodium Hydroxide | PH adjuster |

- **Ceramide 2**^{™}: active ingredient marketed by Sederma, consisting of a pure substance identical to the ceramides present in the skin, which constitute 40 to 50% of the skin lipids. This ingredient has a restructuring function and maintains hydration and integrity of the skin barrier.
- **White Tea^{™}:** active ingredient marketed by Crodarom, based on an extract of *Camellia sinensis,* has both anti-microbial, anti-inflammatory and anti-radical properties.

**4) Smoothing mask cream**

| **Product** | % | **INCI name** | **Role** |
|---|---|---|---|
| **Part A** | | | |
| H₂O | Qsp100 | Water | - |

| **Part B** | | | |
|---|---|---|---|
| Glycerin | 4.00 - 8.00 | Glycerin | Humectant |
| Natrosol 250 M | 0.20-0.50 | Hydroxyethylcellulose | Gelling agent |
| Keltrol CG-SFT | 0.20-0.50 | Xantam Gum | Gelling agent |
| Matguar 5000 | 0.20-0.50 | Cyamopsis Tetragonoloba (Guar) Gum | Gelling agent |
| Phenoxyethanol | Qs | Phenoxyethanol | Preservative |

| **Part C** | | | |
|---|---|---|---|
| Crodafos MCK-SO-(RB) | 1.50 - 3.00 | Potassium Cetyl Phosphate | Surfactant |

| **Part D** | | | |
|---|---|---|---|
| Arlacel 170-PA-(RB) | 5.00 | Glyceryl Stearate & PEG-100 Stearate | Surfactant |
| Crodacol CS90-PA-(RB) | 0.50 - 1.50 | Cetearyl Alcohol | Wax |
| Crodamol AB-LQ-(RB) | 3.00 - 6.00 | C12-15 Alkyl Benzoate | Dry ester |
| Crodamol ISIS-LQ-(MV) | 2.00 - 5.00 | Isostearyl Isostearate | Ester |
| Crodamol STS-LQ-(MH) | 2.00 - 5.00 | PPG-3 Benzyl Ether Myristate | Dry ester |
| Ingredient of the invention | 2.00 | - | Cosmetic active |

| **Part E** | | | |
|---|---|---|---|
| Potassium sorbate | qs | Potassium Sorbate | Preservative |

| **Part F** | | | |
|---|---|---|---|
| Phytessence Stevia GL ^{™} | 1.00 | - | Cosmetic active |

| **Part H** | | | |
|---|---|---|---|
| FruitBio^{™} | 1.00 | AHA | Cosmetic active |

- **Phytessence Stevia GL**^{™}: active ingredient marketed by Crodarom, contains an extract of *Stevia Rebaudiana* which confers moisturizing, soothing, smoothing, antioxidant, anti-fatigue properties.
- **FruitBio**^{™}: active ingredient actif marketed by Sederma, which is a complexe of α-hydroxyacids associated to green tea extract, used for its smoothing property.

**5) Sanitary hydroalcoholic gel**

| **Product** | % | **INCI name** | **Role** |
|---|---|---|---|
| **Part A** | | | |
| H₂O | Qsp100 | Water | Excipient |
| Volarest FL-LQ-(RB) | 2.50 | Acrylates/ Beheneth-25 Methacrylate Copolymer | Rheology agent |
| Potassium sorbate | qs | Potassium Sorbate | Preservative |

| **Part B** | | | |
|---|---|---|---|
| Zemea | 2.00 - 5.00 | Propanediol | Humectant |
| Phenoxyethanol | Qs | Phenoxyethanol | Preservative |
| Crovol A70-LQ-(RB) | 0.50 - 1.50 | PEG-60 Almond Glycerides | Overgreasing agent |

| **Part C** | | | |
|---|---|---|---|
| H₂O | Qs | Water | - |
| NaOH 30 % | Qs | Sodium Hydroxide | |

| **Part D** | | | |
|---|---|---|---|
| Ethanol 96 surfin | 6.00 - 10.00 | Alcohol | Solvent |
| Ingredient of the invention | 2.00 | | Cosmetic active |

| **Part E** | | | |
|---|---|---|---|
| Ecodermine^{™} | 2.00 | - | Cosmetic active |

| **Part F** | | | |
|---|---|---|---|
| Rock Crystal^{™} | 1.00 | - | Cosmetic active |

- **Ecodermine**^{™}**:** active ingredient marketed by Sederma including an association of lactitol and xylitol, aimed to fight skin problems due to microbial imbalance (dryness, itching) by preserving the natural mechanism of skin defense.
- **Rock Crystal**^{™}**:** active ingredient marketed by Crodarom, based on colorless quartz (rock crystal), silicon confers revitalizing, regenerating, protective and remineralizing properties.

### E) In vivo tests

A varnish prepared according to Galenic Example 1) of paragraph D) above was used for these tests. The placebo consists of the same varnish without the active ingredient according to the treatment of the invention.

### Principle

The efficacy evaluation of the varnish according to the invention was carried out on a panel of 25 Caucasian women over the age of 45 having brittle nails.

### Protocol

### Particular inclusion criteria

- To have soft nails.
- To have longitudinal ridges on nails.
- To have brittle/weakened or split nails.
- Do not be pregnant or breastfeeding.
- Be hormonally stable for at least 3 months, and not consider modification during the duration of the test.
- Stop treatment varnish (hardener or conditioner type) 1 month before the start of the test and no longer apply varnish.
- No application of semi-permanent varnish 1 month before the start of the test.
- Stopping treatment creams for nails or cuticles (care oil type) 15 days before the start of the test and apply only a simple moisturizing cream on hands, avoiding nails if necessary.

### Study type and duration

The study was conducted as a single blind versus placebo, volunteers being their own control.

The volunteers had to apply the varnish three times a week on the nails at room temperature (about 10g per application) with removal of the varnish in place with a solvent once a week before applying a new coat.

The study was conducted in single blind versus placebo, volunteers being their own control.

The volunteers had to apply the varnish three times a week on the nails at room temperature (about 10g per application) with removal of the varnish in place with a solvent once a week before applying a new coat.

The synopsis of the study can be summarized according to the following diagram.

| | | |
|---|---|---|
| **T0** | **T 1month** | **T 4months** |
| **Measurements:** | **Measurements:** | **Measurements:** |
| Ridges | Ridges | Ridges |
| Growth rate | | Growth rate |
| Thickness of the nail plate | | Thickness of the nail plate |
| Matrix density | Matrix density | Matrix density |
| Water loss | | Water loss |
| Hardeness | | Hardness |

Various complementary methods and apparatus have been associated for these measurements:

| **Test** | **Apparatus used** |
|---|---|
| Visualization and quantification of ridges | Standardized photographs bench |
| | Fringes projection on prints |
| Quantification of the rate of growth | Standardized photographs bench |
| Visualization and quantification of the plate thickness | Dermatoscopic camera |
| | Echography |
| Visualization and quantification of the matrix density | Echography |
| Quantification of water loss (PIE) | Vapometer |
| Hardness (free edge sampling) | Hardness measurement apparatus |

### Results:

### 1) Improvement of the quality of the nail matrix (its roots):

Compared with placebo, there is an increase in the density of the nail matrix that is synonymous with better quality. This is corroborated by the joint observation of an increase in nail growth rate relative to placebo.

### 2) Improvement of the quality of the nail plate:

Compared to the placebo, there is a decrease in the dryness of the nail (decrease of the water loss), responsible in part for the ridges. This is corroborated by the standardized photos on which a clear decrease of the ridges on the nail is observed (a decrease of their depth compared to the placebo).

### 3) Detailed results

### Nail thickness

### Echography

Using a Cortex ultrasound system equipped with its high-frequency probe, images 6 mm wide by 3 mm thick with sufficient resolution for a good thickness measurement of the tablet were acquired. Three images were extracted and the thickness of the nail measured in the same place with an image analysis tool.

**Table 12: Variation of the nail plate thickness after application of the extract of Apium graveolens according to the invention (N=31 volunteers, n=3 measurements/volunteer).**

| **Echography** | **Nail plate thickness (mm)** | | |
|---|---|---|---|
| | **T0** | **T1 month** | **T4 months** |
| Mean | 0.320 | 0.331 | 0.357 |
| Standard deviation | 0.037 | 0.043 | 0.041 |
| **Variation vs. T0** | | **+3.4%** | **+11.6%** |
| Significance | | *p<0.01* | *p<0.01* |
| Maximum | | 17% | 23% |
| Respondents | | 71% | 90% |

The cosmetic treatment of the invention generated a significant increase in nail thickness of +3.4% in just one month. This increase reached +11.6% (*p<0.01*) after 4 months of application.

### Dermatoscopic camera

A different and complementary measurement of the thickness of the nail was carried out on the free edge of the nail using a dermatoscopic camera. This high-precision camera is equipped with constant LED lighting which ensures perfect standardisation of colors, brightness and geometry. The measuring zone of the edge of the nail was 6 mm x 4 mm. As for the previous study, the thickness of the plate at the edge of the nail was measured by image analysis (photos below).

**Table 13: Variation of the nail plate thickness after application of the extract of Apium graveolens according to the invention (N=25 volunteers).**

| **Echography** | **Nail plate thickness (mm)** | | |
|---|---|---|---|
| | **T0** | **T1 month** | **T4 months** |
| Mean | 0.321 | 0.344 | 0.356 |
| Standard deviation | 0.090 | 0.095 | 0.094 |
| **Variation vs. T0** | | **+7.2%** | **+10.9%** |
| Significance | | *p<0.05* | *p<0.01* |
| Maximum | | 56% | 71% |
| Respondents | | 68% | 68% |

The results observed with this technique confirm those obtained with ultrasound. An increase in thickness by 7.2% (*p <0.05*) after one month and an even better improvement by +10.9% (*p<0.01*) after 4 months of treatment was observed.

The example below shows the measured effect. Interestingly, it was also possible to note on the pictures the change in surface smoothness and the architecture of the nail. After the treatment, the three layers of the nail plate are clearly visible. They appear more dense and transparent, probably due to an increased content in lipids and water.

The two methods used for thickness measurements allow confirming the effect of the treatment of the invention on the nail plate. The numerical results are not exactly the same probably because the zones of measurements are different and the precision of measurements are also different. Nevertheless, the variations are similar: +37 µm by echography and +35 µm for dermatoscopic camera.

### Rehydration, reduction of water loss

Like the skin, the nail is subject to water loss which is more specifically named TOWL (TransOnychial Water Loss). Like the skin, TOWL is essentially sensitive to the quality of the tissue (*stratum corneum,* nail), although the thickness may also play a role.

TOWL measurements were carried out by placing on the nail an apparatus with a cylindrical chamber where the temperature and humidity sensors are located. Three measures were taken.

**Table 14: Variation of the TOWL after application of the extract of Apium graveolens according to the invention (N=27 volunteers, n=3 measurements per site).**

| **VapoMeter^{™}** | **TOWL (g/m²/h)** | | |
|---|---|---|---|
| | **T0** | **T1 month** | **T4 months** |
| Mean | 20.12 | 19.39 | 18.17 |
| Standard deviation | 5.13 | 5.27 | 4.53 |
| **Variation vs. T0** | | **-3.6%** | **-9.7%** |
| Significance | | *ns* | *p<0.05* |
| Maximum | | -43% | -39% |
| Respondents | | 59% | 59% |

The results show that the application of the extract according to the invention initiates in the first month a tendency to decrease TOWL (-3.6%); This trend is confirmed 3 months later, with the decrease markedly accentuated to reach -9.7% (*p<0.05*)*.* These results reflect a better composition, density and structure of the nail plate which better limits the loss of water and then promotes the rehydration of the nail. This prevents "curling" (when the nail curls down) and brings some more aesthetic transparency.

### Evaluation of ridges by an expert panel

A finger restraining system for producing standardized and reproducible images of the nail was used, mounted on a specific photographic bench equipped with a digital camera and a controlled lighting system. Visualization of the ridges was made possible by a slight gloss.

For each volunteer, a series of photos obtained before and after the applications, was submitted to the opinion of 6 experts who had to validate or not the affirmation: "there are fewer ridges after treatment"; Agree, disagree, neither agree nor disagree.

The percentage of positive responses (34%) was clearly and significantly (*p<0.01*) different from the negative responses (10%), which shows a real effect.

### Nail cohesion

The method used was a cutting test, which measures the energy needed to cut a nail. This gives a good idea of the cohesion before and after treatment. The study was carried out using a UMT tribometer on nails taken during the study at T0 and T4 months. The delay between sampling and measurement as well as storage was standardized between trials to avoid bias. To perform these measurements, the thickness and width of each nail were first measured with a digital calliper. Then, each nail was positioned between the cutting blades, the closing of the blades being ensured by the vertical movement of the "block" (weight).

When closing the nail cutter, the normal force is measured and the vertical movement is recorded. It is thus possible to obtain for each measurement the vertical load-movement curve. The energy parameter required for cutting is calculated using the area under the curve by subtracting the nail signal with the nail-free signal. By reducing this energy to the cut surface (thickness and width), a fracture work (Wf), or hardness, is obtained for each nail. A triple measurement was carried out for each nail during the study.

**Table 15: Variation of the nail cohesion after application of the extract of Apium graveolens according to the invention (N=30 volunteers, n=3 measurements/volunteer).**

| **Tribometer** | **Fracture work Wf (kJ/m²)** | |
|---|---|---|
| | **T0** | **T4 months** |
| Mean | 22141 | 23963 |
| Standard deviation | 3764 | 3669 |
| **Variation vs. T0** | | **+8.2%** |
| Significance | | *p<0.07* |
| Maximum | | 89% |
| Respondents | | 63% |

After application of the extract according to the invention, the energy necessary to cut the nail is more important than before the treatment: +8.2% (p<0.07). The calculation of the fracture work takes into account the thickness of the sample so it doesn't interfere. This means that these results are directly in relation to the structure and density of the nail. The treatment of the invention makes the nails more resilient to shocks and brittle.

### 4) Conclusion:

These results show that a cosmetic product comprising the CO₂ supercritical extract of *Apium graveolens* seeds comprising alkyl-phthalide mixture of the invention as the major component, in this case a varnish, is able to repair weakened nails and nails that had lost their beauty. This product can also be used as a preventive measure.

## Claims

1. Cosmetic non-therapeutic topical use of an extract of *Apium graveolens* seeds prepared by CO₂ supercritical extraction for the topical treatment of nails, said extract comprising at least 50% by weight of alkyl-phthalide mixture constituted of sedanenolide, sedanolide and 3-n-butylphthalide.

2. The use according to claim 1, wherein said extract is prepared by a CO₂ supercritical extraction of grinded seeds between 75.10⁵ and 300.10⁵ Pa pressure and between 30 and 80°C.

3. The use according to claim 2, wherein said extract is prepared by CO₂ supercritical extraction under 90.10⁵ Pa of pressure and at 40°C.

4. The use according to anyone of claims 1 to 3, wherein said alkyl phthalides mixture comprises at least 50% of sedanenolide in weight percentage.

5. The use according to anyone of claims 1 to 4, for a treatment of soft, brittle, split nails and/or having longitudinal ridges.

6. The use according to anyone of claims 1 to 5, wherein said plant extract is diluted in a physiologically acceptable medium.

7. The use according to anyone of claims 1 to 6, for a treatment for increasing the rate of growth of nails, for increasing the thickness of the nail plate, for increasing the density of the nail matrix and/or for decreasing the insensible water loss of nails.

8. A varnish product for a cosmetic nail treatment according to anyone of claims 1 to 7 comprising an *Apium Graveolens* seed extract prepared by CO₂ supercritical extraction comprising at least 50% by weight of alkyl-phthalide mixture constituted of sedanenolide, sedanolide and 3-n-butylphthalide as active component in an adapted excipient.

9. The varnish product according to claim 8, wherein said alkyl phthalides mixture comprises at least 50% of sedanenolide in weight percentage.

## Patentansprüche

1. Kosmetische, nicht-therapeutische topische Verwendung eines Extrakts aus *Apium graveolens-*Samen*,* hergestellt durch überkritische CO₂-Extraktion, zur topischen Behandlung von Nägeln, wobei besagter Extrakt mindestens 50 Gew.-% eines Alkylphthalidgemischs, bestehend aus Sedanenolid, Sedanolid und 3-n-Butylphthalid, umfasst.

2. Verwendung nach Anspruch 1, wobei besagter Extrakt durch eine überkritische CO₂-Extraktion gemahlener Samen bei einem Druck zwischen 75,10⁵ und 300,10⁵ Pa und einer Temperatur zwischen 30 und 80 °C hergestellt wird.

3. Verwendung nach Anspruch 2, wobei besagter Extrakt durch überkritische CO₂-Extraktion unter einem Druck von 90,10⁵ Pa und bei 40 °C hergestellt wird.

4. Verwendung nach einem der Ansprüche 1 bis 3, wobei besagtes Alkylphthalidgemisch mindestens 50 % Sedanenolid in Gewichtsprozent umfasst.

5. Verwendung nach einem der Ansprüche 1 bis 4 zur Behandlung von weichen, brüchigen, gespaltenen Nägeln und/oder Nägeln mit Längsrillen.

6. Verwendung nach einem der Ansprüche 1 bis 5, wobei besagter Pflanzenextrakt in einem physiologisch annehmbaren Medium verdünnt ist.

7. Verwendung nach einem der Ansprüche 1 bis 6 zur Behandlung zum Erhöhen der Wachstumsrate von Nägeln, zum Erhöhen der Dicke der Nagelplatte, zum Erhöhen der Dichte der Nagelmatrix und/oder zum Verringern des unmerklichen Wasserverlusts von Nägeln.

8. Lackprodukt für eine kosmetische Nagelbehandlung nach einem der Ansprüche 1 bis 7, umfassend einen *Apium* Graveolens-Samenextrakt, hergestellt durch überkritische CO₂-Extraktion, umfassend mindestens 50 Gew.-% eines Alkylphthalidgemischs, bestehend aus Sedanenolid, Sedanolid und 3-n-Butylphthalid als aktive Komponente in einem geeigneten Hilfsstoff.

9. Lackprodukt nach Anspruch 8, wobei besagtes Alkylphthalidgemisch mindestens 50 % Sedanenolid in Gewichtsprozent umfasst.

## Revendications

1. Utilisation d'un extrait de graines d'*Apium graveolens* obtenu par une extraction au CO₂ supercritique pour un traitement topique des ongles, ledit extrait comprenant au moins 50% en poids d'un mélange d'alkyl-phthalides constitué de sédanénolide, sédanolide et 3-n-butylphthalide.

2. Utilisation selon la revendication 1, **caractérisée en ce que** ledit extrait est obtenu par une extraction au CO₂ supercritique de graines broyées entre 75.10⁵ et 300.10⁵ Pa de pression et entre 30 et 80°C.

3. Utilisation selon la revendication 2, **caractérisée en ce que** ledit extrait est obtenu par une extraction au CO₂ sous une pression de 90.10⁵ Pa et à 40°C.

4. Utilisation selon l'une des revendications 1 à 3, **caractérisée en ce que** le mélange d'alkyl-phthalides comprend au moins 50% de sédanénolide en pourcentage en poids.

5. Utilisation selon l'une des revendications 1 à 4, pour un traitement des ongles mous, cassants, dédoublés et/ou présentant des stries longitudinales.

6. Utilisation selon l'une des revendications 1 à 5, **caractérisé en ce que** ledit extrait de plante est dilué dans un milieu physiologiquement acceptable.

7. Utilisation selon l'une des revendications 1 à 6, pour un traitement permettant d'augmenter la vitesse de pousse des ongles, d'augmenter l'épaisseur de la tablette de l'ongle, d'augmenter la densité de la matrice de l'ongle et/ou de diminuer la perte insensible en eau de l'ongle.

8. Produit de vernis pour le traitement cosmétique des ongles selon l'une quelconque des revendications 1 à 7 comprenant un extrait de graines d*'Apium graveolens* obtenu par une extraction au CO₂ supercritique comprenant au moins 50% en poids d'un mélange d'alkyl-phthalides constitué de sédanénolide, sédanolide et 3-n-butylphthalide comme composant actif dans un excipient adapté.

9. Produit de vernis selon la revendication 8, **caractérisé en ce que** le mélange d'alkyl-phthalides comprend au moins 50% de sédanénolide en pourcentage en poids.
